# EUROPEAN PATENT SPECIFICATION

(11) **EP 1 834 661 B1**
(45) Date of publication and mention of the grant of the patent: **05.08.2009**
(21) Application number: 07002738.8
(22) Date of filing: 08.02.2007
(51) Int. Cl.: A61M 25/00, A61M 25/01

(54) **A stylet device and medical tube assembly**
Eine Mandrin-Vorrichtung und medizinisches Schlauchset
Dispositif de stylet et assemblage d'un tube médical

(30) Priority: 17.03.2006 JP 2006075473
(43) Date of publication of application: 19.09.2007
(73) Proprietor: Covidien AG, 8212 Neuhausen am Rheinfall (CH)
(72) Inventor: Hayakawa, Toshinobu, Fukuroi-shi Shizuoka-ken (JP); Hoshinouchi, Yuya, Fukuroi-shi Shizuoka-ken (JP); Sakai, Yosuke, Fukuroi-shi Shizuoka-ken (JP)
(74) Representative: Tomlinson, Edward James

(56) References cited:
- WO-A-97/31677
- WO-A2-01/72368
- JP-A- 8 089 582
- US-A- 4 207 873
- US-A- 5 396 902
- US-A1- 2003 040 684
- US-A1- 2003 045 831

## Description

### Technical field

The present invention pertains to a stylet device and : an ileus l tube assembly in which the stylet is inserted into : an ileus tube.

### Prior art

A large variety of tubes in the way of medical tubes is available to be inserted into the living body. These are called ileus tubes, which are tubes used for diagnosis and therapy of enterostasis. An ileus tube is inserted transnasally or orally until it reaches the appropriate position in the small intestine, for example, and is used to reduce pressure inside the patient, suction out contents, or inject chemicals such as contrast media, for example. Before reaching the small intestines, for example, the ileus tube must pass through the pyloric ring and Treitz's ligament. Because these areas are curved and narrow, it is not easy for the ileus tube to pass through them. In particular, if the rigidity of the ileus tube is low, sometimes when it passes through the pyloric ring its apical end will abut the stomach wall and bend, thus coiling up within the stomach. Therefore, it has been proposed to insert a stylet into the ileus tube, to increase the rigidity of the tube and allow it to advance through complex passages.

In Japanese Kokai Utility Model No. Sho 63[1988]-77055, the attachment of a weight to the apical end of a medical tube in which a stylet has been inserted is described. The weight makes bending of the apical end of the medical tube possible, and thus the aforementioned medical tube may be advanced smoothly through the passages of bending body cavities. In Japanese Kokoku Patent No. 2000-217926, an invention is described directed to the structure of a stylet handle to be attached to the tip of a rod (stylet) that is inserted into a medical tube.

A steerable electrode catheter assembly is described in US 2003/0045831 A1 having a tip steered by guide wires. WO 01/72368 A2 describes a catheter arrangement for an intralumenal visualization system having a steerable insert. US 5,396,902 describes a steerable stylet steered by a control wire. US 2003/0040684 describes a steerable stylet for use within an intravascular device which is steerable by means of a core wire. WO 97/31677 describes a steerable cardiac instrument having a tip which is steerably by means of a stylet inserted into the instrument. JP 08-089582 describes a catheter arrangement which is steered by the use of magnets on the surface of the body of a patient.

### Summary of the Invention

The present invention refers to an ileus tube assembly wherein a stylet is inserted so that the rigidity of the tube is increased by the rigidity of the stylet., In consequence, the ileus tube does not easily bend even in complex passages, thus allowing advancement of the medical tube within passages. However, in the more complex passages, such as the passages in the vicinity of the pyloric ring and Treitz's ligament mentioned above, the medical tube cannot follow the curvature of passages merely by imparting rigidity to the medical tube by a stylet. Here, the medical tube assembly described in Japanese Kokai Utility Model No. Sho 63-77055provides for the apical end of the medical tube to be bent by a weight, and this is utilized to enable advance through curved passages of internal cavities, thus increasing the possibility of advancement through complex passages as compared to that of a conventional medical tube assembly. Nevertheless, this medical tube assembly requires that the patient's posture is changed for every change in passage curvature direction to match the medical tube curvature orientation with the curvature direction of the passage.

In view of the situation described above, the objective of the present invention is to offer a stylet device to allow easy advancement of an ileus tube through complex passages, and an ileus tube assembly that has an ileus tube with the stylet inserted.

In order to accomplish the objective described above, the stylet device of the present invention is characterized in that it comprises a stylet that is formed in an elongated shape and is suitable to bend or deform, and for which an actuator wire lumen has been formed along the axial direction in a position that is radially biased from the internal axial core, and an actuator wire that is inserted into the aforementioned actuator wire lumen, one end of which is affixed to the aforementioned stylet, and a tension-conferring means that is connected to the other end of the aforementioned actuator wire and that imparts tension to the aforementioned actuator wire. The ileus tube assembly of the present invention is characterized in that it comprises a stylet device having the structure described above, and with an ileus tube, into which the stylet of the stylet device is inserted.

In the aforementioned inventive stylet device, when the tension-conferring means imparts tension to the actuator wire, this tension is transmitted to the stylet to which the actuator wire is affixed. The tension is transmitted to a position deflected from the axial core of the stylet, because the actuator wire lumen, through which the actuator wire has been inserted, has been formed along the axial direction (lengthwise) of the stylet, and also because it has been formed in a position that is radially biased from the axial core of the stylet. Therefore, the stylet bends in the direction in which the actuator wire lumen through which the actuator wire is inserted, has been deflected. Accordingly, by inserting this kind of stylet into an ileus tube, the ileus tube bends according to the above-described bending of the stylet. In this way, not only does the invented stylet device provide rigidity to the ileus tube, but it also enables active bending of the medical tube. Thus it is possible for the apical end of the ileus tube to be deflected with the curvature of the passage, which enables passage of the ileus tube through even complex passages.

The inventive stylet is formed of any material, that is suitable to bend or deform. This "bending deformation" is by any bending method, such as flexure, bending, and curving, for example, as long as the axial core of the stylet deforms into a condition other than that of a straight line. It is sufficient, if only part of the stylet is bendable or deformable, even if the entire stylet is not bendable or deformable.

The inventive stylet device is also characterized in that multiple aforementioned actuator wire lumens are formed in the aforementioned stylet, and the aforementioned actuator wires are inserted through at least two of the multiple aforementioned actuator wire lumens. This enables bending of the stylet in the various directions in which the multiple actuator wires have respectively been imparted with tension. A suitable number of actuator wire lumens through which actuator wires may be inserted is 2-4, formed at intervals of 80°-180° around the circumference of the stylet; they are also formed at equal intervals. In this case, by manipulating multiple actuator wires simultaneously, it is possible to adjust the curvature direction of the stylet and bend the stylet in more directions.

When multiple actuator wires are used, the other end of each actuator wire is connected to a single tension-conferring means. The use of a single tension-conferring means (device) is convenient because this enables pulling and manipulating of the respective actuator wires and thus imparting of tension to them. In this case, the tension-conferring means are structured similar to a multicolor ballpoint pen, for example, or it is structured so that individual actuator levers are individually connected to respective actuator wires; alternatively, the tension-conferring means are characterized in that all the actuator wires are connected to a single actuator lever, and this actuator lever, like a joystick, is freely deflected off-center, thus adjusting the tensile status of the actuator wires.

The inventive stylet is also structured so that it is furnished with an apical end to which one end of the aforementioned actuator wire is affixed, and a main unit that is connected to the aforementioned apical end, with the aforementioned apical end being less rigid than the aforementioned main unit. This makes it possible to bend only the apical end of the stylet when tension is applied to the actuator wire. In order to obtain an apical end that is less rigid than the main unit, the apical end is formed from a softer material than the main unit. For example, a stylet for which only the apical end bends are realized if the apical end is formed of a soft resin and the main unit of a hard resin. When identical material is used for the entire unit, it is still possible to actualize a stylet for which only the apical end bends by causing the outer radius of the apical end to be smaller than that of the main unit, thus lowering the rigidity of the apical end. When bringing about a structure such that only the apical end bends by causing a difference in rigidity or hardness between the apical end portion ("apical end") and the other portions ("main unit") of the stylet, as mentioned above, it is possible for the actuator wire lumen to be formed from the apical end of the stylet towards the main unit.

Another characteristic of the inventive ileus tube assembly is the structure whereby the stylet that is inserted into the ileus tube is furnished with an apical end to which one end of the aforementioned actuator wire is affixed, and with a main unit that is connected to the aforementioned apical end; the aforementioned ileus tube comprises a main tube unit into which the aforementioned main unit is inserted, and with a guidance member that guides the aforementioned main tube unit into cavities within the body, and into which the aforementioned apical end is inserted, such that when tension is imparted to the aforementioned actuator wire by the aforementioned tension-conferring means, the aforementioned guidance member is bent. This structure, wherein the apical end portion (guidance member) of the ileus tube is bent, enables smooth passage through narrow and complex passages.

In this case, the guidance member of the ileus tube is structured to be less rigid than the main tube unit, thus making it possible for only the guidance member to be bent. For example, if the hardness of the guidance member of the ileus tube is less than that of the main tube unit, it is possible for only the guidance member of the ileus tube to be bent. Alternatively, by means of a structure whereby the apical end of the stylet inserted into the : ileus tube is less rigid than the main unit, it is possible to bend only the guidance member.

With regard to the invented : ileus tube assembly, multiple lumens are formed within the : ileus tube, a balloon is attached to its periphery, and a weight is attached to the guidance member.

### Brief description of the figures

Figure 1 is a general view of a : ileus tube assembly of an embodiment of the present invention.
Figure 2 is a cross-sectional view of a main tube unit of the ileus tube of an embodiment of the present invention.
Figure 3 is a longitudinal sectional view of the vicinity of a guidance member of the ileus tube of an embodiment of the present invention.
Figure 4 is a cross-sectional view of a stylet of an embodiment of the present invention.
Figure 5 is a longitudinal sectional view of the vicinity of an apical end of a stylet of an embodiment of the present invention.
Figure 6 is a longitudinal sectional view of a tension-conferring device of an embodiment of the present invention.
Figure 7 is a cross-section at A-A of Figure 6.
Figure 8 is a view showing the condition wherein the apical end of a stylet is bent.
Figure 9 is a view showing the condition wherein the guidance member of an ileus tube is bent.
Figure 10 is a longitudinal sectional view showing the condition wherein the guidance member of an ileus tube is bent.
Figure 11 is a view showing another example of the guidance member of an ileus tube.
Figure 12 is a top view showing another example of a stylet device.
Figure 13 is a side view showing the other example of astylet device.
Figure 14 is a cross-sectional view showing the stylet of the other example of the stylet device.
Figure 15 is a view showing the bent condition of the stylet apical end of the stylet device of the other example.

### Detailed description of preferred embodiments

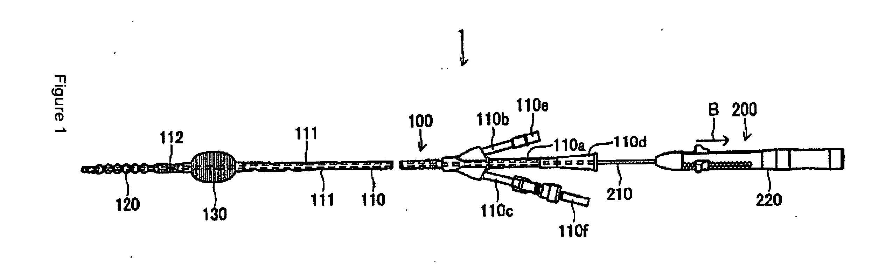

The following statements explain the stylet device and ileus tube assembly of the present invention using drawings. Figure 1 is a front view showing the entire ileus tube assembly of this embodiment. The medical tube assembly 1 has an ileus tube 100 as the medical tube and stylet device 200. Ileus tube 100 comprises a main tube unit 110 and a guidance member 120 wherein the guidance member 120 is on the apical end of main tube unit 110 (shown on the left side of the drawing). When ileus tube 100 is inserted into body cavities transnasally or orally, the guidance member becomes the leading member and advances into the body cavity, and guides main tube unit 110 to the affected area. The main tube unit 110 is a tubular member that follows guidance member 120, within which are formed several lumens that function as corridors when the contents of the affected area are to be drained to the outside, or chemicals are to be supplied to the affected area from the outside, for example. In the left portion of the drawing, balloon 130 has been attached to the circumference of main tube unit 110. This balloon 130 becomes inflated when it receives a supply of fluid from the balloon lumen to be described below, and comes in contact with the inside wall of the small intestines, for example, downstream of the stomach. In the drawing, balloon 130 is shown in its inflated condition, but balloon 130 is contracted when ileus tube 100 is inserted into the body cavity.

The circumference of main tube unit 110 is provided with multiple inlet ports 111. The irrigation side hole 112 is provided on the circumference of main tube unit 110 closer to guidance member 120 than to the part where balloon 130 is attached. The basal end of main tube unit 110 branches into three branches (the first branch 110a, the second branch 110b, and the third branch 110c). The stylet device 200 is provided with a stylet 210 formed in an elongated shape, and a tension-conferring device 220 that is connected to the tip of stylet 210. When ileus tube 100 is inserted into a body cavity, stylet 210 is inserted into ileus tube 100 via first branch 110a as shown in the drawing, and increases the rigidity of ileus tube 100.

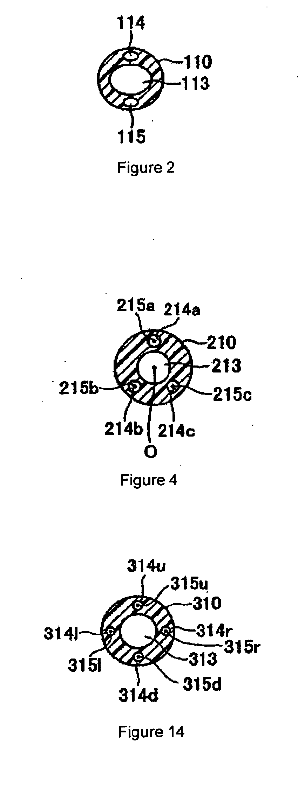

Figure 2 is a radial cross-section of main tube unit 110 of ileus tube 100. As shown in Figure 2, within main tube unit 110 a suction lumen 113 is formed, which is a lumen for draining the contents from within the central part of the body to the outside. The balloon lumen 114, which in the figure is shown above suction lumen 113, is formed to be a lumen for supplying fluid to balloon 130. The irrigation tube 115, which in the figure is shown below suction lumen 113, is formed as a lumen for supplying air to the body cavity in order to prevent negative pressure in the body cavity. The irrigation tube 115 is also used to deliver chemicals, such as contrast medium, into the body. The suction lumen 113 is formed along the axial direction of main tube unit 110. The multiple inlet ports 111 formed on the circumference of main tube unit 110 communicate with suction lumen 113. The balloon lumen 114 is formed along the axial direction of main tube unit 110 parallel to suction lumen 113, and communicates with balloon 130. The irrigation tube 115 is also formed along the axial direction of main tube unit 110, parallel to suction lumen 113. The irrigation side hole 112 formed on the circumference of main tube unit 110 communicates with the irrigation tube 115.

The suction lumen 113 communicates with the first branch 110a that branches from the basal end of main tube unit 110, balloon lumen 114 communicates with the second branch 110b, and irrigation tube 115 communicates with the third branch 110c. As shown in Figure 1, valve 110e is connected to the end of second branch 110b. The backflow valve 110f is connected to the end of third branch 110c. The backflow valve 110f permits fluids to flow from the outside into third branch 110c, but blocks fluids from flowing from third branch 110c to the outside. The connector 110d is attached to the end of first branch 110a. The stylet 210 of stylet device 200 is inserted into first branch 110a via this connector 110d. As can be understood from Figure 1, stylet 210 is formed as an elongated shape with a length that allows for insertion from the apical end of ileus tube 100 towards the posterior end.

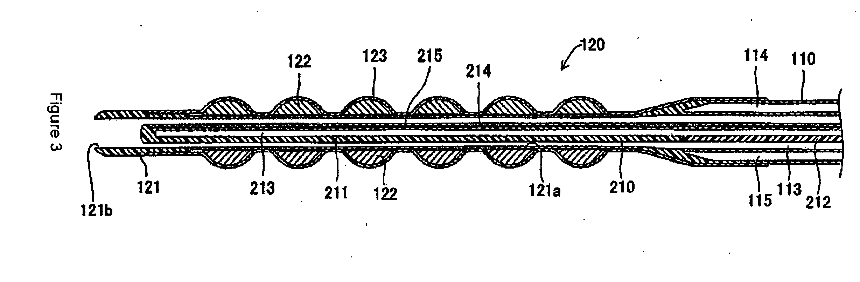

Figure 3 is an axial cross-section showing the vicinity of guidance member 120 of ileus tube 100 when stylet 210 has been inserted. As shown in Figure 3, guidance member 120 comprises guidance tube member 121, which communicates with main tube unit 110, and weights 122, which are attached so as to cover the periphery of guidance tube member 121. The weights 122 present an annular shape in which spheres are perforated by a cylindrical hole. A corridor 121 a is formed within guidance tube member 121. One end of corridor 121a communicates with suction lumen 113 of main tube unit 110, and the other end opens to the outside as apical end aperture 121 b of guidance tube member 121. The weights 122 (six in this example) are attached, allowing for a predetermined interval along the axial direction of guidance tube member 121. The periphery of the weights 122 is covered with a soft resin cover 123, and it is by means of this cover 123 that the axial movement of each weight 122 is regulated, and the weights 122 are fixed in predetermined positions on guidance tube member 121.

The stylet 210 is inserted into first branch 110a from connector 110d, and then inserted from first branch 110a into suction lumen 113 of main tube unit 110, and into corridor 121a of guidance tube member 121 (cf. Figure 3). The stylet 210 has an apical tip 211 and a main unit 212, as shown in Figure 3. The apical tip 211 is formed on the apical end of stylet 210, and is the portion that is inserted into corridor 121 a within guidance tube member 121 of ileus tube 100. The main unit 212 is the part that follows after apical tip 211, and is inserted into suction lumen 113 of main tube unit 110 of ileus tube 100. The apical tip 211 is formed of soft resin (such as soft polyurethane or low-density polyethylene, for example), and main unit 212 is formed of hard resin (such as hard polyurethane or high-density polyethylene, for example). This enables apical tip 211 to bend or deform into a flexion or curve, for example.

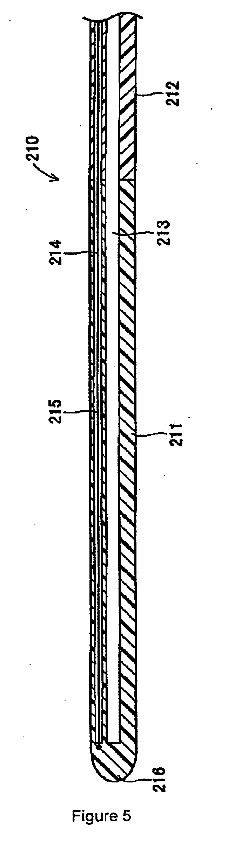

Figure 4 shows a radial cross-section of stylet 210, and, Figure 5 shows an axial cross-section of the vicinity of apical tip 211 of stylet 210. As shown in Figures 4 and 5, stylet 210 is formed such that lumen 213 runs lengthwise through its approximate central region. A reinforcing core wire (omitted from the drawings) is inserted into lumen 213. In addition to reinforcing stylet 210 by inserting a core wire into lumen 213, it is possible to reinforce the stylet by manufacturing the stylet so that a plastic resin covers the core wire.

As shown in Figure 4, stylet 210 has been formed so that multiple (i.e. three in this embodiment) actuator wire lumens 214a, 214b, and 214c (hereinunder collectively referred to in the singular as "actuator wire lumen 214") have been formed so as to surround lumen 213. Each actuator wire lumen 214a, 214b, 214c is formed in a position that is offset from the axial core O of stylet 210. As shown in Figure 5, the actuator wire lumen 214a, 214b, 214c is formed lengthwise from apical tip 211 of stylet 210 towards main unit 212; at one end it is closed in a position close to leading member 216 of stylet 210, while the other end is open from the basal end of stylet 210 (not shown in the drawing). Within each actuator wire lumen 214a, 214b, and 214c, corresponding actuator wires 215a, 215b, and 215c have been inserted (hereinunder collectively referred to in the singular as "actuator wire 215"). As shown in Figure 5, one end of each actuator wire 215a, 215b, 215c is thrust through the closed end of actuator wire lumen 214a, 214b, and 214c, and fixed in stylet 210 in a position close to leading member 216 of stylet 210.

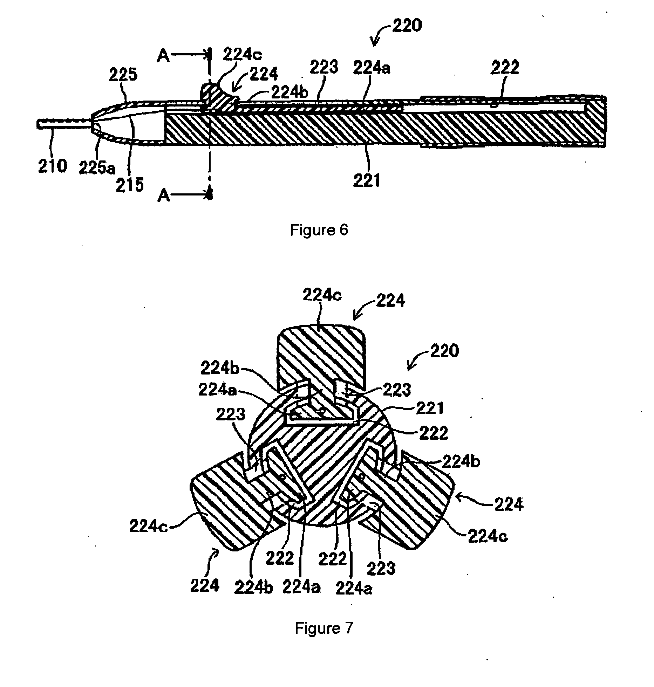

The stylet 210, into which the three actuator wires 215a, 215b, and 215c have been inserted, is connected to the tension-conferring device 220 on the basal end, as shown in Figure 1. In the present embodiment, the tension-conferring device 220 is formed into an elongated shape resembling a ball-point pen. Figure 6 is an axial cross-section across the plane that includes the lengthwise axis of tension-conferring device 220. Figure 7 is an enlarged cross-section at A-A of Figure 6. As shown in these drawings, the tension-conferring device 220 has a main unit 221 formed in a rounded bar shape. Within the main unit 221, three corridors 222 have been formed at equal circumferential intervals. On the periphery of main unit 221, windows 223 have been formed, in which slit-shaped sections have been cut out along the axial direction. Three of these windows 223 are formed at equal circumferential intervals, each communicating with a corridor 222, and opposite that corridor 222, thus creating apertures in a fixed portion of each corridor 222 (in Figure 6, the portion is about one-half). The width of each window 223 is narrower than the width of each corridor 222.

An actuator lever 224 is attached to main unit 221. This actuator lever 224 has a guiding member 224a, coupling member 224b, and an actuator member 224c. The guiding member 224a is inserted into corridor 222, and is able to move in the axial direction of main unit 221 along corridor 222. The coupling member 224b is emplaced vertically on guiding member 224a, extends radially outwards from main unit 221, and projects outwards from window 223. The actuator member 224c is connected to the radially projecting portion of coupling member 224b. Accordingly, guiding member 224a is moveable along window 223, and thereby moveable within corridor 222, by operating actuator member 224c with fingers. Note that three actuator levers 224 have been provided at circumferentially equal intervals on main unit 221, opposite the three corridors 222.

As shown in Figure 6, at the apical end of main unit 221, the cover 225 has been attached. The cover 225 is formed in a bullet shape with a hollow interior, and the end with the larger radius is connected to the end of main unit 221, while the end with the smaller radius forms the aperture 225a. The stylet 210 is attached to aperture 225a. The actuator wire 215 is inserted through actuator wire lumen 214 of stylet 210, and this actuator wire 215 advances from the basal end of stylet 210 into corridor 222 of main unit 221 via the space within cover 225. Its end is affixed to the guiding member 224a of actuator lever 224. Therefore, one end of actuator wire 215 is affixed to apical tip 211 of stylet 210, and the other end is affixed to actuator lever 224. Accordingly, it is possible to impart tension to actuator wire 215, by sliding actuator member 224c to the right, as shown in Figure 6, with a finger, thus pulling the actuator wire 215. It is also possible to provide an energizing means, such as a spring within corridor 222, to energize actuator lever 224; and it is also possible to position actuator member 224c at the left side of Figure 6 at normal times.

When the treatment or diagnosis of ileus is to be conducted with a medical tube assembly 1 of the structure described above, first a condition is created whereby stylet 210 is inserted into ileus tube 100, as shown in Figure 1, and then ileus tube 100 is inserted transnasally or orally with guidance member 120 as the leading side. Then ileus tube 100 passes through the esophagus and reaches the stomach, and as the next step, it is poised to pass through the stomach via the pyloric ring. At this time, in order to pass through the pyloric ring, the operator operates the actuator lever 224 of the tension-conferring device 220, and actuator member 224c moves in the direction of arrow B in Figure 1. This then causes the actuator wire 215, being affixed to the manipulated actuator lever 224, to be pulled. Therefore, the pulled actuator wire 215 becomes tense, and thus tension is imparted to actuator wire 215. This tension is transferred to stylet 210, which is affixed to one end of actuator wire 215. Here, as shown in Figure 4, each of the three actuator wire lumens 214a, 214b, and 214c formed on stylet 210 is arranged in a position that respectively deviates from the axial core of stylet 210, so the tension from the actuator wire is also transmitted to stylet 210, with some displacement from the center of stylet 210. Therefore, stylet 210 tends to contract biased in the direction of an actuator wire lumen (for example, actuator wire lumen 214a) in which an actuator wire (for example, actuator wire 215a) has been inserted to which tension has been imparted, and thus it bends in this direction.

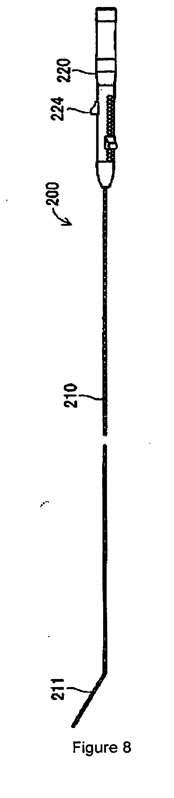

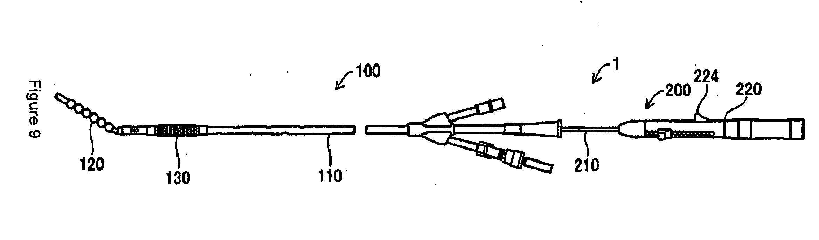

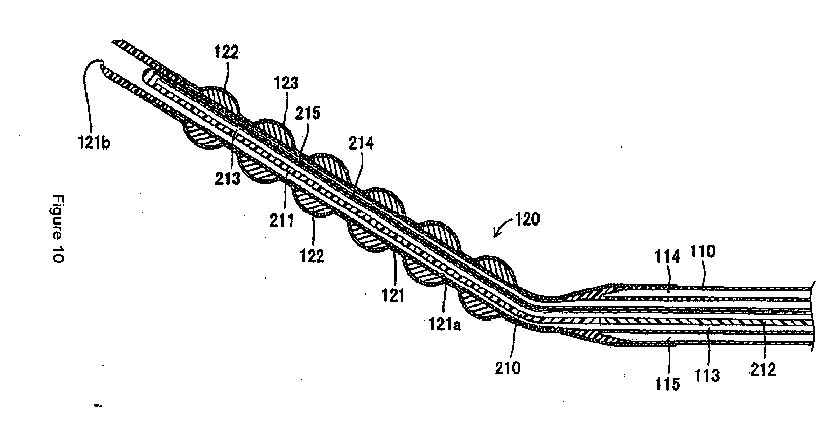

Here, the apical tip 211 of stylet 210 has been formed of soft resin, and the main unit 212 following apical tip 211 has been formed of hard resin, so apical tip 211 is less rigid than main unit 212. Therefore, only apical tip 211 of stylet 210 bends, as shown in Figure 8, due to the aforementioned tension. Because apical tip 211 is bent, as shown in Figure 9, the guidance member 120 of ileus tube 100 in which apical tip 211 has been inserted is bent similarly to apical tip 211. Figure 10 is an axial cross-section of the vicinity of guidance member 120 of ileus tube 100. As shown in Figure 10, the apical tip 211 of stylet 210 curves so as to bend to the side formed by actuator wire lumen 214 in which has been inserted actuator wire 215, to which tension has been imparted. As stylet 210 bends, its apical tip 211 comes into contact with the inner wall of corridor 121a formed in guidance tube member 121 of ileus tube 100, imparting a bending force to ileus tube 100 as well, and thus guidance member 120 of ileus tube 100 is bent. By means of this operation, guidance member 120 is bent in the desired direction, and thus the apical end of guidance member 120 faces the direction of the pyloric ring. In this condition ileus tube 100 is caused to progress further, and is caused to pass through the pyloric ring. Thereafter, byrepeatedly conducting operations identical to those described above, the ileus tube 100 passes through portions having curved passageways, such as Treitz's ligament, for example, and progresses to the small intestine. When the curvature orientation of the passage changes while ileus tube 100 is progressing through the curved passageways, the actuator lever 224 for manipulation is changed to correspond to the curvature orientation. By manipulating two actuator levers 224 simultaneously, it is possible to bend guidance member 120 in a direction intermediate to the flexure directions when lever is manipulated by itself.

When main tube unit 110 of ileus tube 100 reaches the small intestine, for example, the stylet 210 is withdrawn from ileus tube 100. Fluid is supplied from second branch 110b to balloon lumen 114. Then the fluid passes through balloon lumen 114 and is supplied to balloon 130. Balloon 130 becomes enlarged, and comes into contact with the inner wall of the small intestine, for example. The peristaltic movement of the small intestine, for example, is transmitted to balloon 130, and ileus tube 100 then progresses in the small intestine, for example, by the peristaltic movement of the small intestine, for example. Then, the ileus tube 100 progresses to the desired location.

Once the ileus tube 100 has progressed to the target location, a suction bag is connected to first branch 110a. The suction bag is operated, and the inside of suction lumen 113 is depressurized. Then the contents of the small intestines, for example, are taken into suction lumen 113 from apical end aperture 121 b of guidance tube member 121 and inlet ports 111 of main tube unit 110. The contents thus taken in pass from suction lumen 113 through first branch 110a and are recovered in the suction bag. In this way, an ileus, for example, is treated.

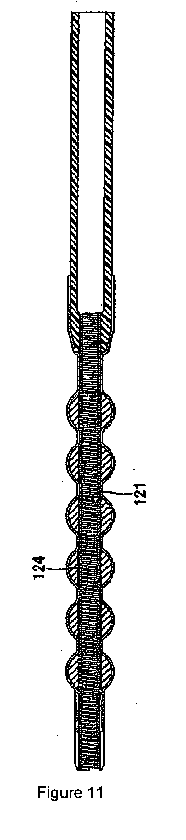

Figure 11 is a drawing showing a different example for the structure of the ileus tube guidance member. In this medical tube, a spring 124 is provided inside guidance tube member 121. Such a structure makes it possible to give more elasticity to guidance tube member 121, and thus enables flexible bending of the entire body of guidance tube member 121.

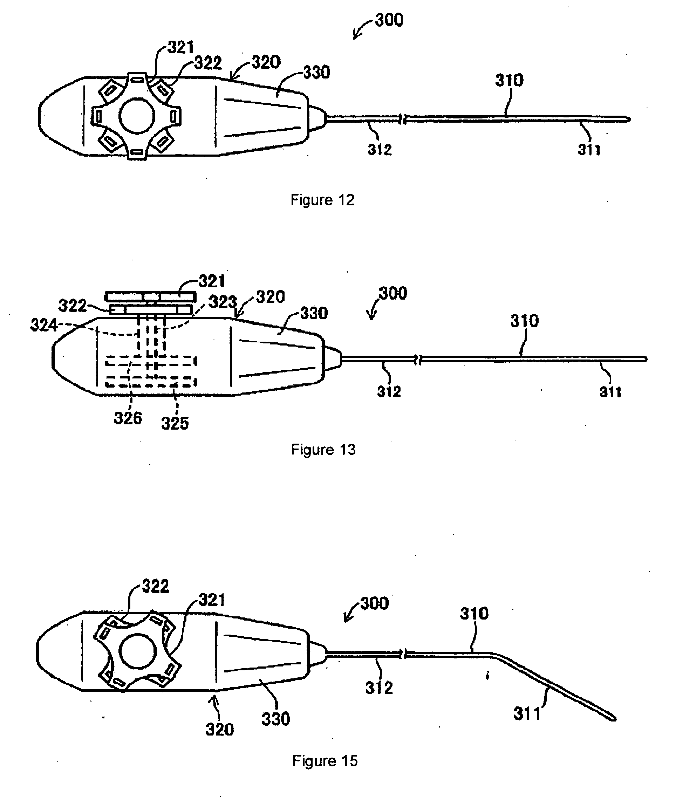

Figure 12 is a top view of another example of the stylet device, and Figure 13 is a side view. This stylet device 300 has a tension-conferring device 320 provided with two rotatable actuator levers (first actuator lever 321, and second actuator lever 322). The first and second actuator levers 321 and 322 are attached one above the other such that they share the same axis of rotation. As shown in Figure 13, the first actuator lever 321 is linked to first disk plate 325 positioned within main unit 330, via first connecting shaft 323, and is structured with first connecting shaft 323 as the axis, so as to rotate as an integrated unit with first disk plate 325. The second actuator lever 322 is linked to second disk plate 326 positioned within main unit 330, via second connecting cylinder 324, and is structured with second connecting cylinder 324 representing the axis to rotate as an integrated unit with second disk plate 326. The second actuator lever 322, second connecting cylinder 324, and the second disk plate 326 are interpolated with first connecting shaft 323, and this assembly is structured to be disposed with first connecting shaft 323 representing the axis. It is possible to permit mutually independent rotation of both plates by, for example, disposing bearings and the like between first disk plate 325 and second disk plate 326.

The stylet 310 has an apical tip 311 formed of soft resin and a main unit 312 formed of hard resin; the main unit 330 of tension-conferring device 320 is attached to the basal end of main unit 312. A radial cross-section of stylet 310 is shown in Figure 14. As shown in Figure 14, lumen 313, into which a reinforcing core wire (omitted from the drawing) is inserted, is formed in the cross-sectional center of stylet 310, and four actuator wire lumens are formed at 90° intervals on the circumference of lumen 313. In the present embodiment, the actuator wire lumen formed on the right side of the drawing is right lumen 314r, the actuator wire lumen formed on the left side is left lumen 314l, the actuator wire lumen formed on the top side is upper lumen 314u, and the actuator wire lumen formed on the lower side is lower lumen 314d. The actuator wire lumen 314d, 3141, 314r, 314u is formed along the axial orientation of stylet 310 from apical tip 311 towards main unit 312. The actuator wires 315r, 3151, 315u, and 315d have been inserted into actuator wire lumens 314r, 3141, 314u, and 314d. One end of actuator wire 315r, 3151, 315u, 315d is affixed to apical tip 311 of stylet 310 and passes insertionally through actuator wire lumen 314d, 314l, 314r, 314u, and the other end is affixed to first disk plate 325 or second disk plate 326.

Attached to disk plates 325 and 326, seen from the radial cross-section of stylet 310 (Figure 14), are the ends of actuator wires 315r, 3151, 315u, 315d, which are inserted into lumens 314d, 3141, 314r, 314u, in positions opposite lumen 313. For example, actuator wires 315r and 3151 inserted into right lumen 314r and left lumen 3141 may be affixed to first disk plate 325, and actuator wires 315u and 315d inserted into upper lumen 314u and lower lumen 314d may be affixed to second disk plate 326. The actuator wires 315r, 315l, 315u, 315d are affixed to disk plates 325 and 326 such that, when disk plate 325 or 326 is rotated in a single direction, one of the actuator wires 315 will be pulled, and if it is rotated in the other direction, the other actuator wire 315 will pulled.

Given the structure, described above, when first actuator lever 321 is rotationally manipulated, tension will be imparted to either the actuator wire 315r or actuator wire 315l inserted into right lumen 314r or left lumen 3141, and the apical tip 311 of stylet 310 will bend in the left or right direction (cf. Figure 15). On the other hand, if second actuator lever 322 is rotationally manipulated, tension will be imparted to either the actuator wire 315u or actuator wire 315d inserted into upper lumen 314u or lower lumen 314d, and the apical tip 311 of stylet 310 will bend in the upward or downward direction. If, on the other hand the first and second actuator levers 321 and 322 are simultaneously rotationally manipulated, it is possible to bend the apical end of stylet 310 towards the upper left, or towards the lower right.

As explained above, stylet devices 200 and 300 of the present embodiments are formed in an elongated shape, and are capable of bending deformation; they are furnished with stylets 210 and 310 in which are formed actuator wire lumens 214a, 214b, 214c and 314d, 314l, 314r, 314u along the axial direction in positions that are radially offset from the internal axial cores; with actuator wires 215a, 215b, 215c and 315r, 3151, 315u, 315d that are inserted into actuator wire lumens 214a, 214b, and 214c and 314d, 3141, 314r, 314u, having one end affixed to the apical tips 211 and 311 of stylets 210 and 310; and with tension-conferring devices 220 and 320 that impart tension to actuator wires 215a, 215b, 215c, and 315r, 3151, 315u, 315d and that are connected to the other ends of actuator wires 215a, 215b, 215c and 315r, 3151, 315u, 315d. The apical tips 211 and 311 of stylets 210 and 310 are structured so as to actively bend according to the operation of tension-conferring devices 220 and 320. This makes it possible to actively bend the guidance member 120 of ileus tube 100, into which stylets 210 and 310 have been inserted. Accordingly, it is possible to easily pass ileus tube 100 through even complex passages in the body cavity.

As for stylet 210, the apical tip 211 is formed from a soft resin, whereas the main unit 212 is formed from a hard resin, so apical tip 211 is not as hard as main unit 212. This allows bending or deformation of only apical tip 211. Since only the apical tip 211 of stylet 210 is bent, it is possible to bend only the guidance member 120 of ileus tube 100 in which apical tip 211 is inserted.

The foregoing statements have described working modes of the present invention, but the present invention is not limited to these embodiments. For example, in the aforementioned embodiment, apical tip 211 was less rigid than main unit 212 of stylet 210, in order that only the guidance member 120 of ileus tube 100 would bend, but it is also possible for guidance member 120 of ileus tube 100 to be less rigid than main tube unit 110 in order for only the guidance member 120 to bend. In this case, guidance tube member 121 may be formed from a soft resin, and main tube unit 110 may be formed from a hard resin, thus making it possible for guidance member 120 to be less rigid than main tube unit 110. It is also possible to form guidance tube member 121 and main tube unit 110 out of the same material, but for the external diameter of guidance tube member 121 to be smaller than that of main tube unit 110, and thereby for guidance member 120 to be less rigid than main tube unit 110. Also, in the aforementioned embodiment, in order for apical tip 211 to be less rigid than the main unit 212 of stylet 210, the apical tip 211 was formed of a soft resin while main unit 212 was formed of a hard resin, but it is also possible for the apical tip 211 to be less rigid than main unit 212 by using other materials. It is also possible by means other than changing the material, for example, by changing the shape that the external diameter of apical tip 211 is smaller than the external diameter of main unit 212, so that apical tip 211 is less rigid than main unit 212. Also with regard to the provision of lumen 213 close to the center of stylet 210 and the insertion of a core wire within this lumen 213, the diameter of the core wire that is inserted into the portion of lumen 213 formed within apical tip 211 may be made smaller than the diameter of the core wire that is inserted into the portion that is formed within main unit 212, and thus the rigidity of apical tip 211 will be less than that of main unit 212.

## Claims

1. An ileus tube assembly comprisng a stylet device (200, 300) and an ileus tube (100) having an interior into which a stylet (210, 310) of the stylet device is inserted the stylet device comprising:
a deformable stylet (210, 310) having an elongated shape comprising a distal end part connected to a main part (212, 312) and being provided with an actuator wire lumen (214, 214a, 214b, 214c, 314) extending in an axial direction in a position that is radially off-center with respect to a cross section of said stylet;
an actuator wire (215, 215a, 215b, 215c, 315) inserted into the actuator wire lumen, wherein one end of said actuator wire is affixed to the stylet;, and
a tension-conferring means (220, 320) connected to another end of the actuator wire for imparting tension to the actuator wire,
the ileus tube(100) comprising:
a main tube unit (110) into which the stylet is inserted, and
a guidance member (120) attached to a distal end of the main tube unit for guiding the tube unit into cavities within the body, and into which the distal end part of the stylet is inserted, such that when tension is imparted to the actuator wire by the tension-conferring means (220, 320), the guidance member is bent,
**characterized in that** multiple lumens are formed within the ileus tube (100), a balloon (130) is attached to its periphery, and a weight (122) is attached to the guidance member (120).

2. An ileus tube assembly according to Claim 1, **characterized in that** multiple actuator wire lumens (214, 214a, 214b, 214c, 314) are formed in the stylet (210, 310), and actuator wires (215, 215a, 215b, 215c, 315) are inserted into two or more of the multiple actuator wire lumens.

3. An ileus tube assembly according to Claim 1 or 2, **characterized in that** one end of the actuator wire (215, 215a, 215b, 215c, 315) is affixed to said distal end part of the stylet, and wherein the distal end part is less rigid than the main part.

4. An ileus tube assembly (1) according to Claim 1, **characterized in that** the guidance member (120) is less rigid than the main tube unit (110).

5. An ileus tube assembly (1) according to Claim 1, **characterized in that** the main tube unit (110) comprises a suction lumen (113), a balloon lumen (114) and an irrigation tube (115).

## Patentansprüche

1. Ileusröhrenanordnung mit einer Mandrinvorrichtung (200, 300) und einer Ileusröhre (100), in deren Innenraum ein Mandrin (210, 310) der Mandrinvorrichtung eingeführt wird, wobei die Mandrinvorrichtung Folgendes umfasst:
einen verformbaren Mandrin (210, 310) mit einer länglichen Gestalt und einem distalen Endteil, der mit einem Hauptteil (212, 312) verbunden ist, und mit einem Betätigungsdrahtlumen (214, 214a, 214b, 214c, 314), das sich in einer axialen Richtung in einer relativ zu einem Querschnitt des Mandrins radial außermittigen Stellung erstreckt;
einen Betätigungsdraht (215, 215a, 215b, 215c, 315), der in das Betätigungsdrahtlumen eingeführt wird, wobei ein Ende des Betätigungsdrahts am Mandrin befestigt ist; und
ein Spannungsübertragungsmittel (220, 320), das mit einem anderen Ende des Betätigungsdrahts verbunden ist, um Spannung auf den Betätigungsdraht auszuüben, wobei die Ileusröhre (100) Folgendes umfasst:
eine Hauptröhreneinheit (110), in die der Mandrin eingeführt wird; und
ein Führungselement (120), das an einem distalen Ende der Hauptröhreneinheit befestigt ist, um die Röhreneinheit in Hohlräume im Körper zu führen, und in das der distale Endteil des Mandrins eingeführt wird, so dass das Führungselement gebogen wird, wenn vom Spannungsübertragungsmittel (220, 320) Spannung auf den Betätigungsdraht aufgebracht wird,
**dadurch gekennzeichnet, dass** mehrere Lumen in der Ileusröhre (100) geformt sind, ein Ballon (130) an ihrem Umfang befestigt ist und ein Gewicht (122) am Führungselement (120) befestigt ist.

2. Ileusröhrenanordnung nach Anspruch 1, **dadurch gekennzeichnet, dass** mehrere Betätigungsdrahtlumen (214, 214a, 214b, 214c, 314) im Mandrin (210, 310) geformt sind und Betätigungsdrähte (215, 215a, 215b, 215c, 315) in zwei oder mehr der mehreren Betätigungsdrahtlumen eingeführt sind.

3. Ileusröhrenanordnung nach Anspruch 1 oder 2, **dadurch gekennzeichnet, dass** ein Ende des Betätigungsdrahts (215, 215a, 215b, 215c, 315) am distalen Endteil des Mandrins befestigt ist und worin der distale Endteil weniger starr ist als der Hauptteil.

4. Ileusröhrenanordnung (1) nach Anspruch 1, **dadurch gekennzeichnet, dass** das Führungselement (120) weniger starr ist als die Hauptröhreneinheit (110).

5. Ileusröhrenanordnung (1) nach Anspruch 1, **dadurch gekennzeichnet, dass** die Hauptröhreneinheit (110) ein Sauglumen (113), ein Ballonlumen (114) und eine Irrigationsröhre (115) umfasst.

## Revendications

1. Ensemble de tube pour ileus, comprenant un dispositif de stylet (200, 300) et un tube pour ileus (100) ayant un intérieur dans lequel un stylet (210, 310) du dispositif de stylet est inséré, le dispositif de stylet comprenant :
un stylet déformable (210, 310) ayant une forme allongée comprenant une partie d'extrémité distale connectée à une partie principale (212, 312) et
pourvue d'une lumière pour fil actionneur (214, 214a, 214b, 214c, 314) s'étendant dans une direction axiale dans une position radialement décentrée par rapport à une section transversale dudit stylet ;
un fil actionneur (215, 215a, 215b, 215c, 315) inséré dans la lumière pour fil actionneur, une extrémité dudit fil actionneur étant attachée au stylet ; et
un moyen de tensionnement (220, 320) connecté à une autre extrémité du fil actionneur pour tendre le fil actionneur, le tube pour ileus (100) comprenant :
une unité de tube principale (110) dans laquelle est inséré le stylet ; et
un organe de guidage (120) attaché à une extrémité distale de l'unité de tube principale pour guider l'unité de tube dans des cavités dans le corps, et dans lequel la partie d'extrémité distale du stylet est insérée, de telle sorte que lorsqu'une tension est appliquée au fil actionneur par le moyen de tensionnement (220, 320), l'organe de guidage soit courbé,
**caractérisé en ce que** de multiples lumières sont formées dans le tube pour ileus (100), un ballonnet (130) est attaché à sa périphérie et un poids (122) est attaché à l'organe de guidage (120).

2. Ensemble de tube pour ileus selon la revendication 1, **caractérisé en ce que** de multiples lumières pour fil actionneur (214, 214a, 214b, 214c, 314) sont formées dans le stylet (210, 310), et des fils actionneurs (215, 215a, 215b, 215c, 315) sont insérés dans deux ou plus des multiples lumières pour fil actionneur.

3. Ensemble de tube pour ileus selon la revendication 1 ou 2, **caractérisé en ce qu'**une extrémité du fil actionneur (215, 215a, 215b, 215c, 315) est attachée à ladite partie d'extrémité distale du stylet, la partie d'extrémité distale étant moins rigide que la partie principale.

4. Ensemble de tube pour ileus (1) selon la revendication 1, **caractérisé en ce que** l'organe de guidage (120) est moins rigide que l'unité de tube principale (110).

5. Ensemble de tube pour ileus (1) selon la revendication 1, **caractérisé en ce que** l'unité de tube principale (110) comprend une lumière d'aspiration (113), une lumière pour ballonnet (114) et un tube d'irrigation (115).
